# EUROPEAN PATENT APPLICATION

(11) **EP 3 100 686 A1**
(43) Date of publication of application: **07.12.2016**
(21) Application number: 15742852.5
(22) Date of filing: 23.01.2015
(51) Int. Cl.: A61B 17/00, A61B 1/00

(54) **MEDICAL DEVICE AND MEDICAL SYSTEM**

(30) Priority: 29.01.2014 JP 2014013918
(71) Applicant: Olympus Corporation, Tokyo 192-8507 (JP)
(72) Inventor: KIKUCHI, Chisato, Hachioji-shi, Tokyo 192-8507 (JP); KISHI, Kosuke, Hachioji-shi, Tokyo 192-8507 (JP); YOSHII, Toshihiro, Hachioji-shi, Tokyo 192-8507 (JP)
(74) Representative: Schicker, Silvia
(86) International application number: PCT/JP2015/051782
(87) International publication number: WO 2015/115317

(57) **Abstract**

An object of the invention is to provide a medical apparatus and a medical system with an improved operability.

The medical apparatus 1 is characterized by including an endoscope 2, a manipulator 3 having at least one degree of freedom, a flexible cylindrical sheath 4 mounted on an outer circumference of the manipulator 3, a treatment tool 5 inserted through the sheath 4, and an endoscope/manipulator operating unit 11 for independent operation of the endoscope 2 and manipulator 3.

## Description

### Technical Field

The present invention relates generally to a medical apparatus and a medical system including a treatment tool inserted in a patient's body for viewing and treating an affected site.

### Background Art

In recent years, there has been laparoscopic surgery carried out with a manipulator inserted in a patient's body for treatment of affected sites. Laparoscopic surgery is applied to a small incision cut open in the patient's abdomen, so there are less burdens on the patient. However, multiple treatment tools are used for the surgery and handled in a limited body cavity, resulting in the need for improving on their operability.

Patent Publication 1 discloses how to attach a treatment tool to an endoscope using an external tube in such a way as not to reduce the flexibility of a flexible portion.

### Citation List

### Patent Literature

Patent Publication 1: JP(A) 5-307143

### Summary of Invention

### Technical Problem

With the prior art of Patent Publication 1 wherein the endoscope is integral with the treatment tool, however, it is likely that the field of view of the endoscope may be obstructed by the treatment tool. In addition, the endoscope cannot be operated separately from the treatment tool because they always work together.

One embodiment according to the invention has been contrived with the abovementioned object(s) in mind, and provides a medical apparatus and a medical system improved in terms of operability.

### Solution to Problem

According to one embodiment of the invention, there is a medical apparatus provided, characterized by including:
an endoscope,
a manipulator having at least one degree of freedom,
a flexible cylindrical sheath mounted on an outer circumference of the manipulator,
a treatment tool inserted through the sheath, and
an endoscope/manipulator operating unit for independent operation of the endoscope and the manipulator.

According to one embodiment of the invention, the medical apparatus further includes an endoscope/manipulator control unit for independent control of the endoscope and the manipulator, wherein:
the endoscope/manipulator operating unit includes an endoscope operating unit and a manipulator operating unit separately, and
the endoscope/manipulator control unit includes an endoscope control unit and a manipulator control unit separately.

According to one embodiment of the invention, the medical apparatus further includes:
a first connector member for connecting together the manipulator and the sheath, and
a second connector member located adjacent to
   the first connector member for connecting together the manipulator and the sheath, wherein the length of the sheath located between the adjoining first and second connector members is longer than the length of the manipulator between the adjoining first and second connector members along an axial direction of the manipulator.

In the medical apparatus according to one embodiment of the invention, the first or second connector member connects together the manipulator and the sheath such that they are relatively movable.

According to one embodiment of the invention, the medical apparatus further includes a movement restrictor for stopping movement of the first or second connector members in a given position for movement restriction.

According to one embodiment of the invention, the medical apparatus further includes a fixation mechanism for temporal fixation in a given position of the treatment tool which can move forward and backward into from the sheath.

In the medical apparatus according to one embodiment of the invention, the manipulator further includes an end effector for implementing medical treatments.

In the medical apparatus according to one embodiment of the invention, the treatment tool and the end effector are independently operable.

In the medical apparatus according to one embodiment of the invention, the treatment tool is capable of fixing the manipulator temporarily in place, and the manipulator is capable of fixing the treatment tool temporarily in place.

According to one embodiment of the invention, there is a medical system provided, characterized by including:
a system control unit for control of a medical apparatus, and
a display unit for displaying images acquired through an endoscope, wherein the system control unit enables images acquired through the endoscope to be shown on the display unit.

### Advantageous Effects of Invention

With the medical apparatus and medical system described herein, it is possible to implement operation compatible with each treatment thereby improving on operability.

### Brief Description of Drawings

Fig. 1 is illustrative in schematic of one example of the medical apparatus according to the first embodiment of the invention.
Fig. 2 is illustrative in schematic of one example of the medical apparatus according to the second embodiment of the invention.
Fig. 3 is illustrative in schematic of one example of the medical apparatus according to the third embodiment of the invention.
Fig. 4 is illustrative in schematic of one example of the medical apparatus according to the fourth embodiment of the invention.
Fig. 5 is illustrative in schematic of one example of the medical apparatus according to the 5^{th} embodiment of the invention.
Fig. 6 is illustrative in schematic of one example of the medical apparatus according to the 6^{th} embodiment of the invention.
Fig. 7 is illustrative in schematic of one example of the medical apparatus according to the 7^{th} embodiment of the invention.
Fig. 8 is illustrative in schematic of one example of the medical apparatus according to the 8^{th} embodiment of the invention.
Fig. 9 is illustrative in schematic of one example of attachment of the manipulator to the sheath in the embodiment described herein.
Fig. 10 is illustrative in schematic of one example of the connector member in the embodiment described herein.
Fig. 11 is illustrative in schematic of one example of the manipulator having a curving part in the embodiment described herein.
Fig. 12 is illustrative in schematic of one example of the manipulator attachment area in the embodiment described herein.
Fig. 13 is illustrative in schematic of one example of the movement restrictor in the embodiment described herein.
Fig. 14 is illustrative in schematic of another example of the movement restrictor in the embodiment described herein.
Fig. 15 is illustrative in schematic of the first actuation state of the end effector and treatment tool in the embodiment described herein.
Fig. 16 is illustrative in schematic of the second actuation state of the end effector and treatment tool in the embodiment described herein.
Fig. 17 is illustrative in schematic of the third actuation state of the end effector and treatment tool in the embodiment described herein.
Fig. 18 is illustrative in schematic of the fourth actuation state of the end effector and treatment tool in the embodiment described herein.
Fig. 19 is illustrative in schematic of the fifth actuation state of the end effector and treatment tool in the embodiment described herein.
Fig. 20 is illustrative in schematic of the sixth actuation state of the end effector and treatment tool in the embodiment described herein.
Fig. 21 is illustrative in schematic of the first example of the fixation mechanism for temporal fixation of the treatment tool to the sheath in the embodiment described herein.
Fig. 22 is illustrative in schematic of the second example of the fixation mechanism for temporal fixation of the treatment tool to the sheath in the embodiment described herein.
Fig. 23 is illustrative in schematic of the third example of the fixation mechanism for temporal fixation of the treatment tool to the sheath in the embodiment described herein.
Fig. 24 is illustrative in schematic of the fourth example of the fixation mechanism for temporal fixation of the treatment tool to the sheath in the embodiment described herein.
Fig. 25 is illustrative in schematic of the fifth example of the fixation mechanism for temporal fixation of the treatment tool to the sheath in the embodiment described herein.
Fig. 26 is illustrative in schematic of the sixth example of the fixation mechanism for temporal fixation of the treatment tool to the sheath in the embodiment described herein.
Fig. 27 is illustrative in schematic of the seventh example of the fixation mechanism for temporal fixation of the treatment tool to the sheath in the embodiment described herein.
Fig. 28 is illustrative of the medical system to which the medical apparatus according to the embodiment described herein is applied.
Fig. 29 is illustrative in architecture of the medical system to which the medical apparatus according to the embodiment described herein is applied.

### Description of Embodiments

Some embodiments of the invention are now explained.

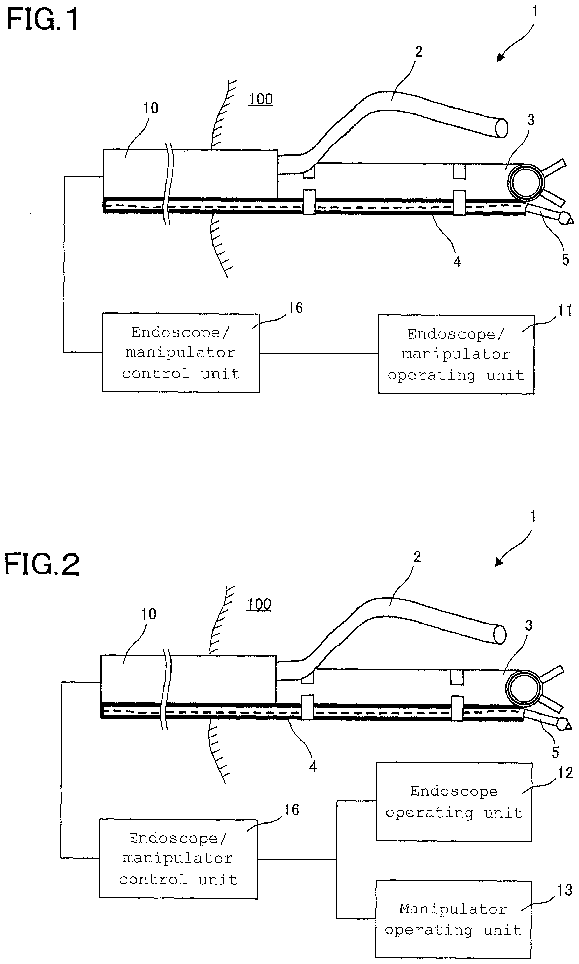

Fig. 1 is illustrative in schematic of one example of the medical apparatus 1 according to the first embodiment of the invention.

In the medical apparatus 1 according to the first embodiment, an endoscope 2 and a manipulator 3 are inserted through a common tubular member 10 having flexibility. The medical apparatus 1 according to the first embodiment includes the endoscope 2, the manipulator 3 having at least one degree of freedom, a flexible cylindrical sheath 4 mounted on the outer circumference of the manipulator 3, a treatment tool 5 inserted through the sheath 4, an endoscope/manipulator operating unit 11 for independent operation of the endoscope 2 and manipulator 3, and an endoscope/ manipulator control unit 16 for controlling the endoscope 2 and manipulator 3 independently in association with operation of the endoscope/manipulator operating unit 11.

As compared with an arrangement wherein the sheath 4 is attached to the endoscope 2, therefore, the treatment tool 5 can more easily be placed within the field of view of the endoscope 2 by mounting the sheath 4 having the treatment tool 5 inserted through it on the manipulator 3 and operating the endoscope 2 or manipulator 3, thereby improving on operability.

The endoscope/manipulator operating unit 11, formed in an integral form, operates the endoscope 2 and manipulator 3 independently, and the endoscope/manipulator control unit 16, formed in an integral form, controls the endoscope 2 and manipulator 3 independently.

Thus, the medical apparatus 1 according to the first embodiment of the invention is well compatible with treatment implemented by independent operation of the endoscope 2 and manipulator 3, resulting in an improved operability.

Fig. 2 is illustrative in schematic of one embodiment of the medical apparatus 1 according to the second embodiment of the invention.

In the medical apparatus 1 according to the second embodiment of the invention, an endoscope operating unit 12 for operation of the endoscope 2 and a manipulator operating unit 13 for operation of the manipulator 3 are used in place of the endoscope/manipulator operating unit 11 in the medical apparatus 1 according to the first embodiment of the invention: they are provided separately and operated independently. In the medical apparatus 1 according to the second embodiment of the invention, an endoscope/manipulator control unit 16 is integrally formed to control the endoscope 2 and manipulator 3 independently.

Thus, the medical apparatus 1 according to the second embodiment of the invention is well compatible with treatment implemented by independent operation of the endoscope 2 and manipulator 3, resulting in a more improved operability.

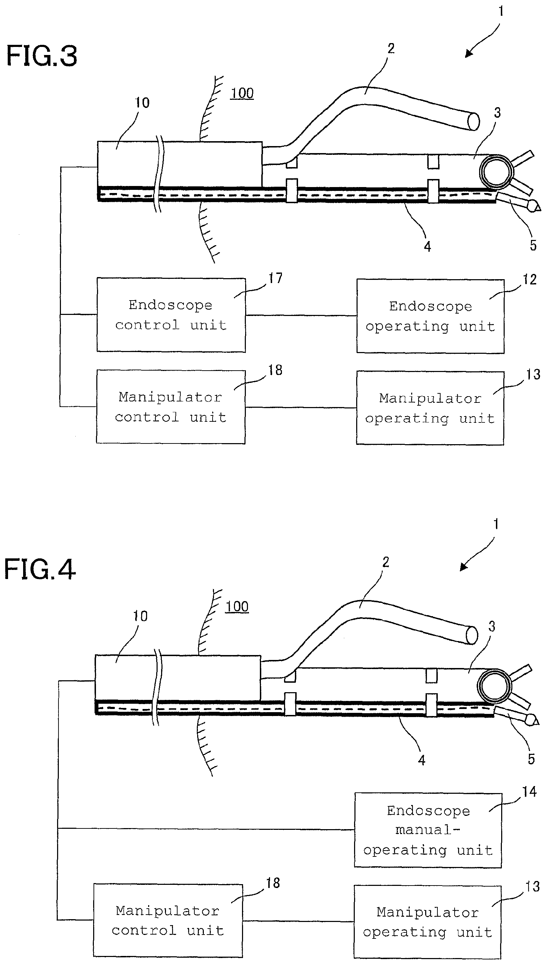

Fig. 3 is illustrative in schematic of one example of the medical apparatus 1 according to the third embodiment of the invention.

In the medical apparatus 1 according to the third embodiment of the invention, an endoscope operating unit 12 for operation of the endoscope 2 and a manipulator operating unit 13 for operation of the manipulator 3 are used in place of the endoscope/manipulator operating unit 11 in the medical apparatus 1 according to the first embodiment of the invention: they are provided separately and operated independently. In the medical apparatus 1 according to the third embodiment of the invention, an endoscope control unit 17 and a manipulator control unit 18 are used in place of the endoscope/manipulator control unit 16 in the medical apparatus 1 according to the first embodiment of the invention are separately provided for independent control of the endoscope 2 and manipulator 3.

Thus, the medical apparatus 1 according to the third embodiment of the invention is well compatible with treatment implemented by independent operation of the endoscope 2 and manipulator 3, resulting in an improved operability.

Fig. 4 is illustrative in schematic of one example of the medical apparatus 1 according to the fourth embodiment of the invention.

In the medical apparatus 1 according to the fourth embodiment of the invention, an endoscope manual-operating unit 14 for manual operation of the endoscope 2 and a manipulator operating unit 13 for operation of the manipulator 3 are used in place of the endoscope/ manipulator operating unit 11 in the medical apparatus 1 according to the first embodiment of the invention: they are provided separately and operated independently. The medical apparatus 1 according to the fourth embodiment of the invention includes only a manipulator control unit 18 for independent control of the manipulator 3 because the endoscope 2 is manually operated.

Thus, the medical apparatus 1 according to the fourth embodiment of the invention is well compatible with treatment implemented by independent operation of the endoscope 2 and manipulator 3, resulting in an improved operability.

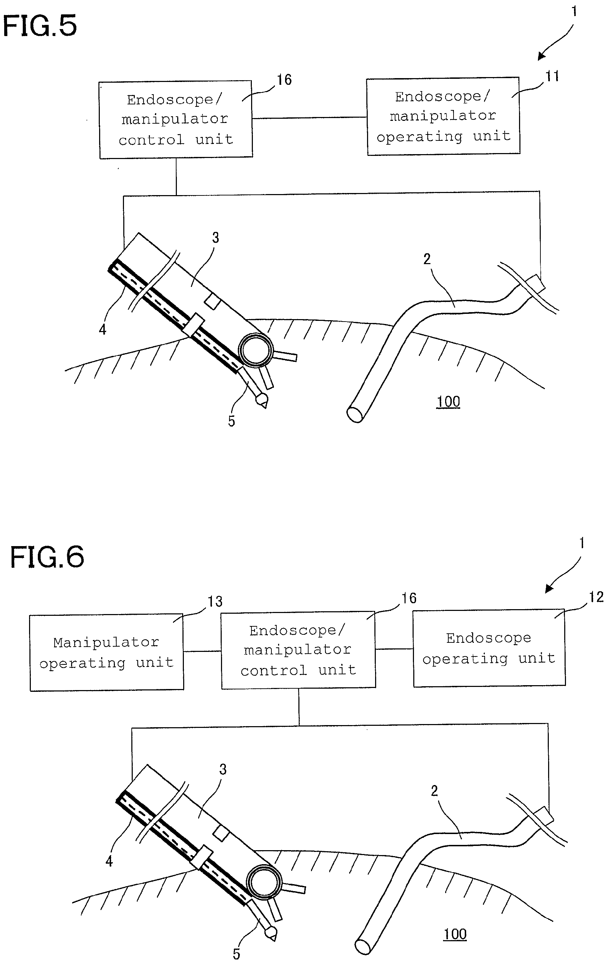

Fig. 5 is illustrative in schematic of one example of the medical apparatus 1 according to the fifth embodiment of the invention.

In the medical apparatus 1 according to the fifth embodiment of the invention, the endoscope 2 is provided separately from the manipulator 3. The medical apparatus 1 according to the fifth embodiment of the invention includes an endoscope 2, a manipulator 3 having at least one degree of freedom, a flexible cylindrical sheath 4 mounted on the outer circumference of the manipulator 3, a treatment tool 5 inserted through the sheath 4, an endoscope/manipulator operating unit 11 for independent operation of the endoscope 2 and manipulator 3, and an endoscope/manipulator control unit 16 for independent control of the endoscope 2 and manipulator 3 in association with operation of the endoscope/manipulator operating unit 11.

The endoscope/manipulator operating unit 11, provided in an integral form, operates the endoscope 2 and manipulator 3 independently, and the endoscope/manipulator control unit 16, provided in an integral form, controls the endoscope 2 and manipulator 3 independently.

Thus, the medical apparatus 1 according to the fifth embodiment of the invention is well compatible with treatment implemented by independent operation of the endoscope 2 and manipulator 3, resulting in an improved operability.

Fig. 6 is illustrative in schematic of one example of the medical apparatus 1 according to the sixth embodiment of the invention.

In the medical apparatus 1 according to the sixth embodiment of the invention, an endoscope operating unit 12 for operation of the endoscope 2 and a manipulator operating unit 13 for operation of the manipulator 3 are used in place of the endoscope/manipulator operating unit 11 in the medical apparatus 1 according to the fifth embodiment of the invention: they are provided separately and operated independently. In the medical apparatus 1 according to the sixth embodiment of the invention, an endoscope/manipulator control unit 16, provided in an integral form, controls the endoscope 2 and manipulator independently.

Thus, the medical apparatus 1 according to the sixth embodiment of the invention is well compatible with treatment implemented by independent operation of the endoscope 2 and manipulator 3, resulting in an improved operability.

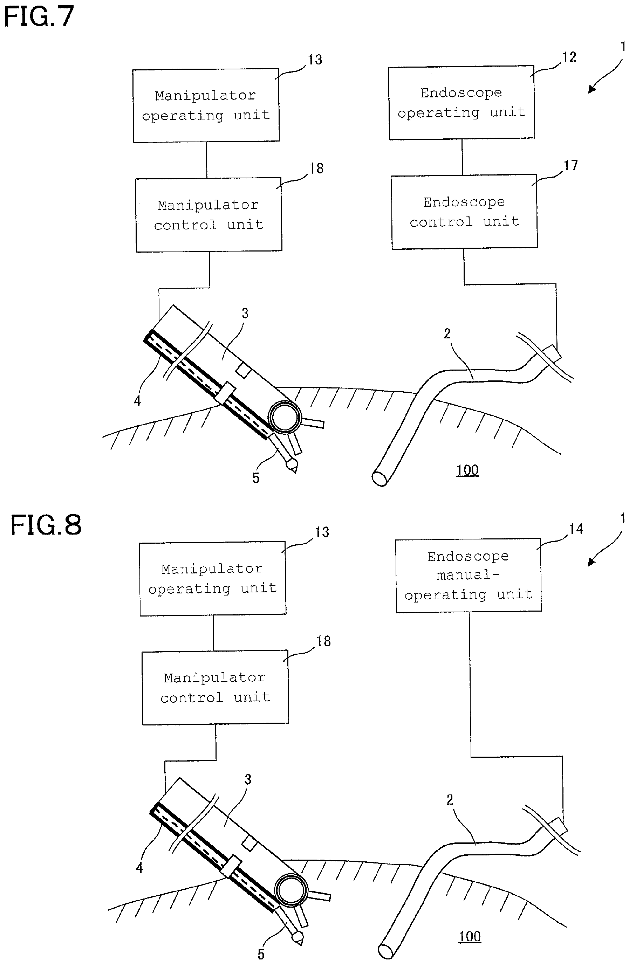

Fig. 7 is illustrative in schematic of one example of the medical apparatus 1 according to the seventh embodiment of the invention.

In the medical apparatus 1 according to the seventh embodiment of the invention, an endoscope operating unit 12 for operation of the endoscope 2 and a manipulator operating section unit 13 for operation of the manipulator 3 are used in place of the endoscope/manipulator operating unit 11 in the medical apparatus 1 according to the fifth embodiment of the invention: they are provided separately and operated independently. In the medical apparatus 1 according to the seventh embodiment of the invention, an endoscope control unit 17 and a manipulator control unit 18 are used in place of the endoscope/manipulator control unit 16 in the medical apparatus 1 according to the fifth embodiment of the invention: they are separately provided for independent control of the endoscope 2 and manipulator 3.

Thus, the medical apparatus 1 according to the seventh embodiment of the invention is well compatible with treatment implemented by independent operation of the endoscope 2 and manipulator 3, resulting in an improved operability.

Fig. 8 is illustrative in schematic of one example of the medical apparatus 1 according to the eighth embodiment of the invention.

In the medical apparatus 1 according to the eighth embodiment of the invention, an endoscope manual-operating unit 14 for manual operation of the endoscope 2 and a manipulator operating unit 13 for operation of the manipulator 3 are used in place of the endoscope/ manipulator operating unit 11 in the medical apparatus 1 according to the fifth embodiment of the invention: they are provided separately and operated independently. The medical apparatus 1 according to the eighth embodiment of the invention includes only a manipulator control unit 18 for independent control of the manipulator 3 because the endoscope 2 is manually operated.

Thus, the medical apparatus 1 according to the eighth embodiment of the invention is well compatible with treatment implemented by independent operation of the endoscope 2 and manipulator 3, resulting in an improved operability.

Attachment of the manipulator 3 to the sheath 4 is now explained.

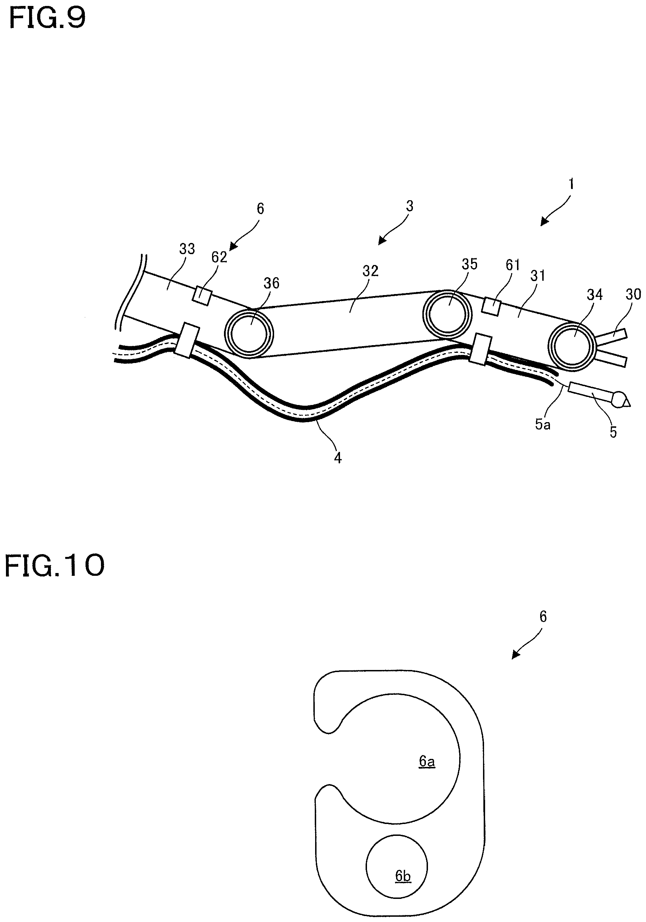

Fig. 9 is illustrative in schematic of one example of how to attach the manipulator 3 to the sheath 4 in the embodiment described herein, and Fig. 10 is illustrative in schematic of one example of a connector member 6.

The manipulator 3 includes an end effector 30, a first arm 31, a second arm 32, a third arm 33, a distal-end actuator 34, a first coupler 35, and a second coupler 36.

The end effector 30 plays a role of the treatment tool for implementing medical treatments such as grasping forceps, local injection needles, electric scalpels or snares. As an end effector operating unit (not shown) is operated by an operator, it causes the end effector 30 to be actuated by the distal-end actuator 34. The distal-end actuator 34 is supported at one end of the first arm 31.

Incorporation of the end effector 30 in the manipulator 3 allows the number of replacement of the treatment tool 5 to be decreased and the time needed for replacement to be shortened. Movement of both the end effector 30 and the treatment tool 5 is enabled by the single manipulator 3 so that both the end effector 30 and the treatment tool 5 can rapidly move to an affected site, ending up with an improvement of the operability of the manipulator 3.

The first coupler 35 couples together the first and second arms 31 and 32 for relative rotation, and the second coupler 36 couples together the second and third arms 32 and 33 for relative rotation. In turn, this makes the first and second arms 31 and 32 bendable at the first coupler 35 and the second and third arms 32 and 33 bendable at the second coupler 36.

The sheath 4 has a cable 5a of the treatment tool 5 inserted through it, and is attached to the manipulator 3 by a connector member 6. As with the end effector 30, the treatment tool 5 is a member for implementing medical treatments such as grasping forceps, local injection needles, electric scalpels or snares. While a part of the treatment tool 5 is made up of an operating cable 5a, it is to be understood that it may be made up of a flexible tubular member. Assume here that the treatment tool 5 takes the form of holding forceps as an example. An opening/closing operating wire is placed in the tubular member. As shown in Fig. 10, a part of the outer circumference of the connector member 6 is cut out to form a notch acting as a manipulator mount 6a, and a round hole acting as a sheath mount 6b is formed in the vicinity of the manipulator mount 6a. The manipulator 3 is fitted into the manipulator mount 6a of the connector member 6, and the sheath 4 is inserted through the sheath mount 6b.

It is here to be noted that the manipulator mount 6a may be formed of a round hole while the sheath mount 6b may be formed of a partially cut out hole. Alternatively, both may be formed of round holes or partially cut out holes.

According to the embodiment described herein, the connector member 6 includes a first connector member 61 attached to the first arm 31 and a second connector member 62 attached to the third arm 33. For this reason, the separation between the first connector member 61 and the second connector member 62 is longer than the distance between a first coupler 35 that defines the distal end of the bending portion and a second coupler 36 that defines the proximal end of the bending portion, and the length of the sheath 4 interposed between the first connector member 61 and second connector member 62 is longer than the length of the manipulator 3 between the adjoining first 61 and second connector member 62 along its axial direction.

Consequently, a portion of the sheath 4 between the first connector member 61 and the second connector member 62 comes to have a play enough to bend the manipulator 3 smoothly between the first connector member 61 and the second connector member 62 without interference of the sheath 4 with bending movement of the manipulator 3, resulting in an improved operability.

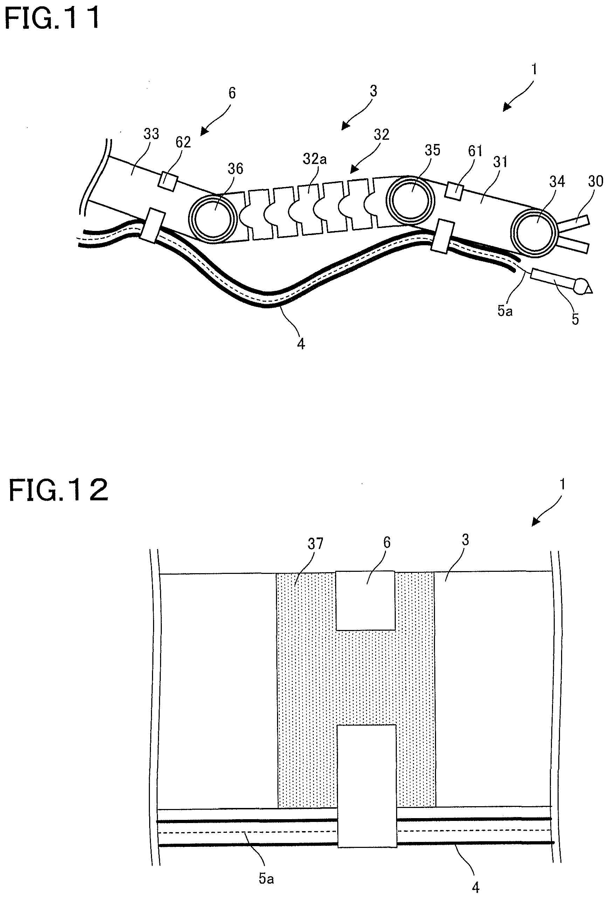

Fig. 11 is illustrative in schematic of one example of the manipulator 3 having a curving part according to the embodiment described herein.

As shown in Fig. 11, a second arm 32 of the manipulator 3 may be made up of a curving arm including a plurality of coupled articulating pieces 32a. When the second arm 32 is made up of a curving arm too, a portion of the sheath 4 between the connector members 6 comes to have a play enough to bend the manipulator 3 smoothly between the connector members 61 and 62 without interference of the sheath 4 with bending movement of the manipulator 3, resulting in an improved operability.

How the connector member 6 is mounted in place is now explained.

The connector members 6 may be mounted in such a way as to be movable toward at least one of the manipulator 3 and sheath 4. It is typically preferable that the connector members 6 are movable in at least one of circumferential and axial directions with respect to the manipulator 3 or sheath 4. Alternatively, at least one of multiple connector members 6 may be movable. Referring typically to the example of Fig. 9, the first connector member 61 of the first arm 31 may be fixed in place while the second connector member 62 of the third arm 33 may be movable. A sliding bearing or the like may be used for such a movable structure.

Thus, as the connector members 6 are movable relative to the manipulator 3, it allows the sheath 4 to move to a position where no active load is applied to it during bending or curving of the manipulator 3 so that even with disturbance like friction, the manipulator 3 can move smoothly.

Fig. 12 is illustrative in schematic of one example of a mounting area 37 of the manipulator 3 in the embodiment described herein.

In the embodiment described herein, the mounting area 37 of the manipulator 3, to which the connector members 6 are attached, may be of reduced friction, as shown in Fig. 12. Alternatively, the mounting area 37 (not shown) of the sheath 4, to which the connector members 6 are attached, may be of reduced friction. In addition, a portion of contact of the connector members 6 with the manipulator 3 may be of reduced friction. The mounting area 37 may have been coated with a low-friction material such as fluororesin.

By way of such low-friction contact of the connector members 6 with the manipulator 3 or sheath 4, the sheath 4 can move to a position where no more active load is applied to it during the bending or curving of the manipulator 3 so that even with disturbance like friction, the manipulator 3 can move more smoothly.

There may be a movement restrictor provided so as to restrict movement of the connector members 6 to a given distance or angle. For instance, when the diameter of the mounting area 37 is smaller than the manipulator 3 adjacent to it, there is a step having a varying diameter left as the movement restrictor that restricts movement of the connector members 6 to a given amount of distance.

In the embodiment described herein, there may further be movement restrictors 38 and 39 provided so as to restrict movement of the connector members 6 such that the movement of the connector members 6 comes to a halt in a given position for movement restriction. In the embodiment described herein, it is also possible to stop movement of the connector members 6 with respect to the manipulator 3. For instance, this facilitates insertion of the cable 5a through the sheath 4.

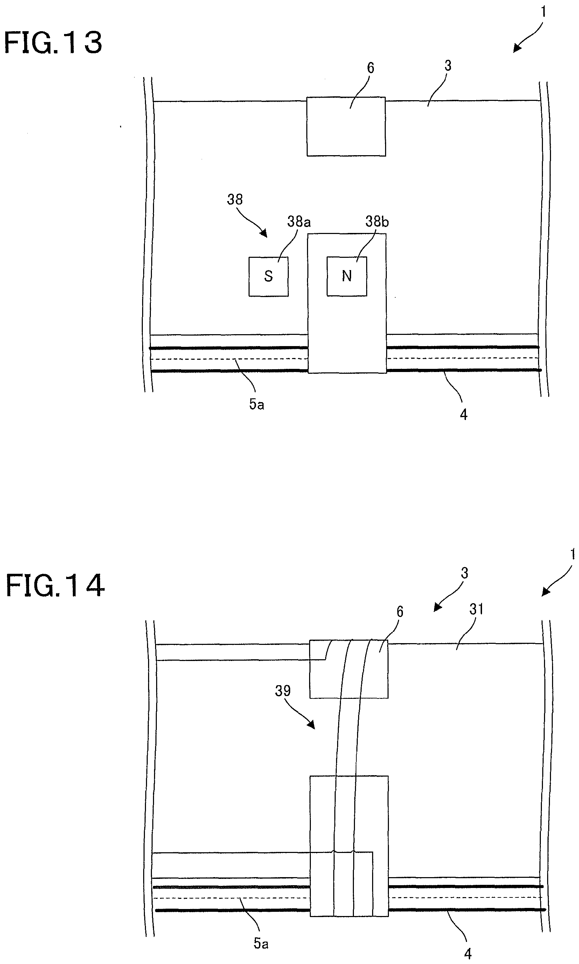

Fig. 13 is illustrative in schematic of one example of the movement restrictor in the embodiment described herein.

In the example of a movement restrictor 38 shown in Fig. 13, electromagnets are located in given positions such that one of the manipulator 3 and connector member 6 becomes an S-pole 38a and the other an N-pole 38b. And with the intention of stopping movement of the connector member 6 with respect to the manipulator 3, an electric current is passed through the electromagnets such that they attract each other, thereby stopping movement of the connector member 6 with respect to the manipulator 3. For instance, this facilitates insertion of the cable 5a through the sheath 4. Note here that a permanent magnet may be used for one magnet.

Fig. 14 is illustrative in schematic of another example of the movement restrictor according to the embodiment described herein.

In the example of the movement restrictor 39 of Fig. 14, a wire or other similar material is wound around the outer circumference of the connector member 6. And with the intention of stopping the connector member 6 with respect to the manipulator 3, the wire may be pulled thereby stopping movement of the connector member 6 with respect to the manipulator 3. For instance, this facilitates insertion of the cable 5a through the sheath 4.

Actuation of the end effector 30 and treatment tool 5 in the manipulator 3 is now explained. In the medical apparatus 1 according to the embodiment described herein, two surgical members: end effector 30 and treatment tool 5 may be used. It is thus possible to reduce the number of replacement of the treatment tool 5 and, hence, shorten the time needed for replacement. Both the end effector 30 and the treatment tool 5 may be operated by the single manipulator 3. It is therefore possible to achieve rapid movement of both the end effector 30 and the treatment tool 5 to an affected site, resulting in an improvement in the operability of the manipulator 3.

When the end effector 30 and treatment tool 5 in the manipulator 3 are individually used, they are preferably actuated, as mentioned below, such that they do not interfere with each other.

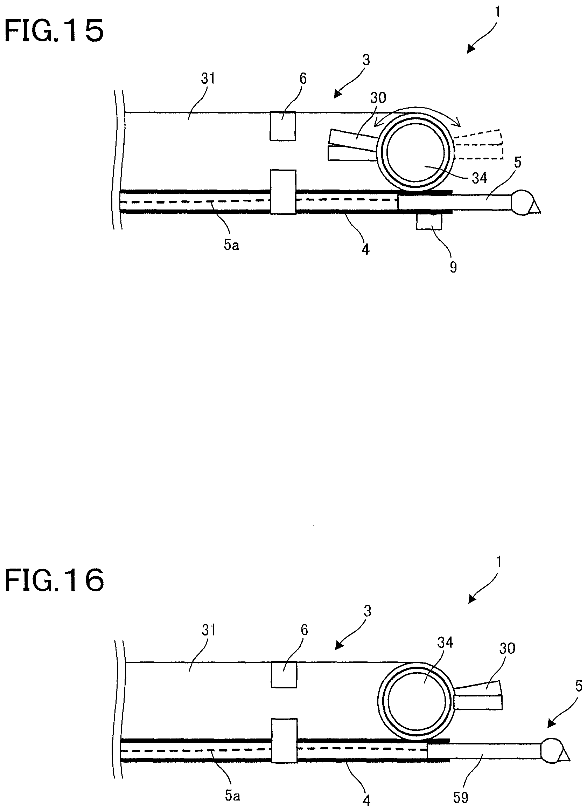

Fig. 15 is illustrative in schematic of the first actuation state of the end effector 30 and treatment tool 5 in the embodiment described herein.

In the first actuation state of the medical apparatus 1 according to the embodiment described herein, only the treatment tool 5 is used. In order to prevent interference of the end effector 3 with the treatment tool 5 in this case, a distal-end actuator 34 is actuated to rotate the end effector 30 to the first arm 31 side. It is here to be noted that a sensor 9 may be provided for detection of whether the treatment tool 5 is found or not. When the sensor 9 detects the presence of the treatment tool 5, the end effector 30 is rotated to the first arm 31 side, and when the sensor 9 does not, the end effector 30 is rotated to the opposite side. While the sensor 9 may be located anywhere in the sheath 4, it is preferably located in the vicinity of the distal end. For the sensor 9, use may be made of a transmission sensor, an electrostatic capacity sensor, a proximity sensor or the like.

In the first actuation state of the medical apparatus 1 according to the embodiment described herein, there is thus no interference of the end effector 30 with actuation of the treatment tool 5 as the end effector 30 of the manipulator 3 is rotated to the first arm 31 side.

Fig. 16 is illustrative in schematic of the second actuation state of the end effector 30 and treatment tool 5 in the embodiment described herein.

In the second actuation state of the medical apparatus 1 according to the embodiment described herein, only the treatment tool 5 is used. To prevent interference of the treatment tool 5 with the end effector 30 in this case, it is preferable that the distal end of the treatment tool 5 extends out from the end effector 30 even with a part of the hard portion 59 of the treatment tool 5 stored in the sheath 4.

In the second actuation state of the medical apparatus 1 according to the embodiment described herein, there is thus no interference of the end effector 30 with actuation of the treatment tool 5 as the treatment tool 5 extends out from the end effector 30.

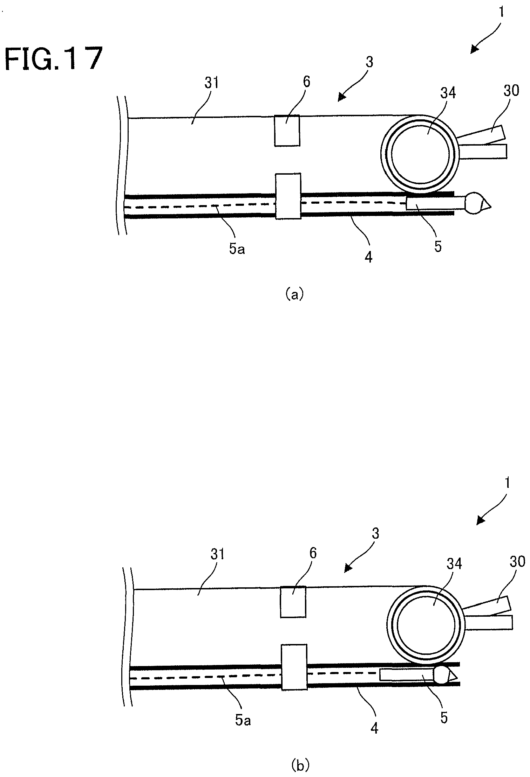

Fig. 17 is illustrative in schematic of the third actuation state of the end effector 30 and treatment tool 5 in the embodiment described herein.

In the third actuation state of the medical apparatus 1 according to the embodiment described herein, only the end effector 30 is used. To prevent interference with actuation of the end effector 30 in this case, the cable 5a is pulled to store the treatment tool 5 within the sheath 4. Upon storing, a part of the treatment tool 5 may be received within the sheath 4 as depicted in Fig. 17(a) or, alternatively, the treatment tool 5 may be received within the sheath 4 in its entirety, as depicted in Fig. 17(b).

In the third actuation state of the medical apparatus 1 according to the embodiment described herein, there is thus no interference of the end effector 30 with actuation of the treatment tool 5 as the treatment tool 5 is stored within the sheath 4.

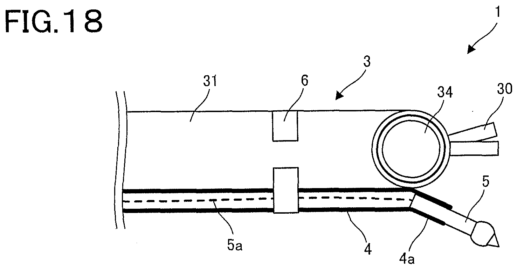

Fig. 18 is illustrative in schematic of the fourth actuation state of the end effector 30 and treatment tool 5 in the embodiment described herein.

In the fourth actuation state of the medical apparatus 1 according to the embodiment described herein, the end effector 30 and the treatment tool 5 are selectively used. In order to prevent interference of actuation of the end effector 30 with actuation of the treatment tool 5 in this case, a guide 4a is formed over the sheath 4 to extend the treatment tool 5 in such a direction as to space away from the end effector 30.

In the fourth actuation state of the medical apparatus 1 according to the embodiment described herein, there is thus no interference of the end effector 30 with the treatment tool 5 upon actuation as the treatment tool 5 extends out in such a direction as to space away from the end effector 30.

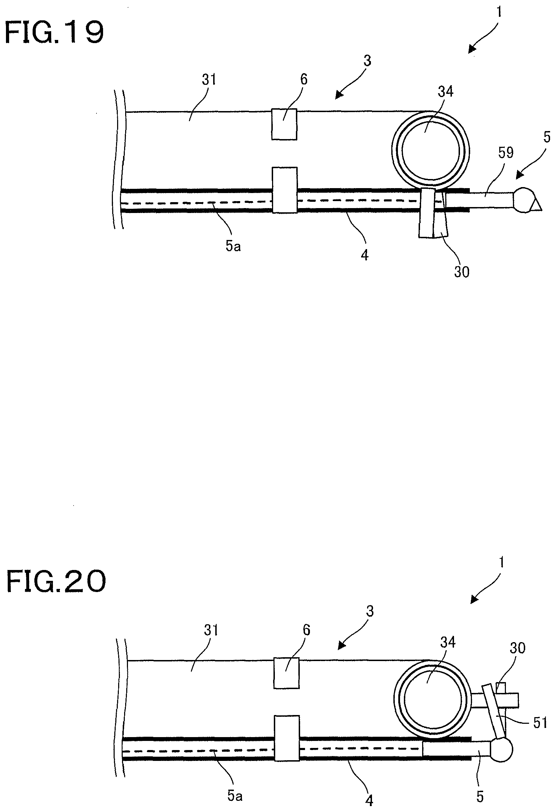

Fig. 19 is illustrative in schematic of the fifth actuation state of the end effector 30 and treatment tool 5 in the embodiment described herein.

In the fifth actuation state of the medical apparatus 1 according to the embodiment described herein, only the treatment tool 5 is used. In the fifth actuation state, a grasping member is used as the end effector 30 to fix the sheath 4 by means of the end effector 30 in place. Note here that both the sheath 4 and the treatment tool 5 stored within the sheath 4 may be fixed by means of the end effector 30 in place.

In the fifth actuation state of the medical apparatus 1 according to the embodiment described herein, there is thus stable actuation of the treatment tool 5 achieved as the sheath 4 or treatment tool 5 is grasped by the end effector 30.

Fig. 20 is illustrative in schematic of the sixth actuation state of the end effector 30 and treatment tool 5 in the embodiment described herein.

In the sixth actuation state of the medical apparatus 1 according to the embodiment described herein, actuations of the end effector 30 and treatment tool 5 are individually restricted. In the sixth actuation state, a grasping member 51 is used as the treatment tool 5, and the end effector 30 is grasped by means of the treatment tool 5.

In the sixth actuation state of the medical apparatus 1 according to the embodiment described herein, the end effector 30 is thus grasped by the treatment tool 5 whereby the actuations of the end effector 30 and treatment tool 5 are individually restricted with the result that there can be reduced interference with other endoscope or manipulator.

The fixation mechanism for temporal fixation of the treatment tool 5 to the sheath 4 in the medical apparatus 1 according to the embodiment described herein is now explained. In the medical apparatus 1 according to the embodiment described herein, the treatment tool 5 is designed such that it is advanceable and retracable the sheath 4. However, there is the need for using the treatment tool 5 while fixed in place; for instance, there is increasing demand for actuation accuracy of the treatment tool 5 during use. It is then possible to fix the treatment tool 5 temporarily to the sheath 4 thereby improving on operability.

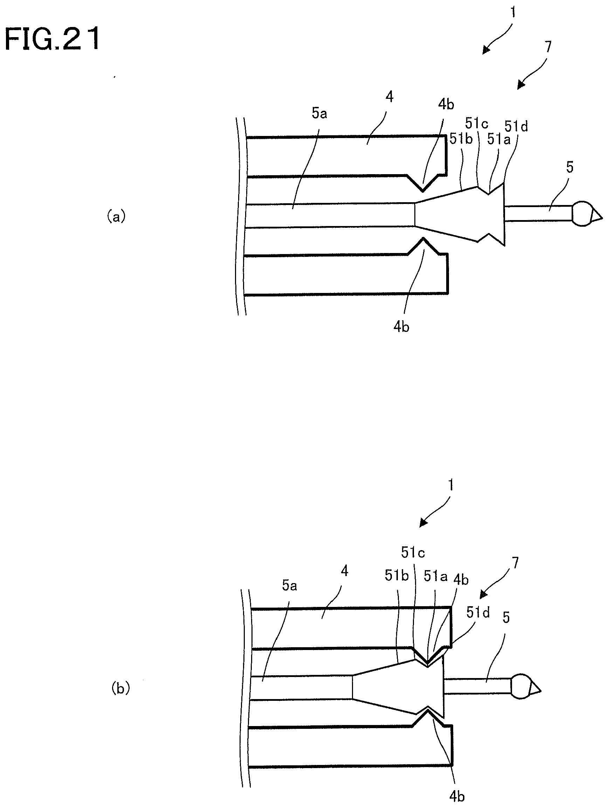

Fig. 21 is illustrative in schematic of one example of the fixation mechanism 7 for temporal fixation of the treatment tool 5 to the sheath 4 in the embodiment described herein.

In the fixation mechanism 7 according to the first example, the sheath 4 includes an inwardly extending convexity 4b, as depicted in Fig. 21(a). The treatment tool 5 includes a concavity 51a fitted over the convexity 4b. On the side of the cable 5a with respect to the concavity 51a, the treatment tool 5 includes a diameter-increasing portion 51b whose diameter increases as it comes close to the convexity 51a, a maximal portion 51c where the diameter of the portion 51b reaches a maximum and a lid 51d formed on the distal end side of the concavity 51a and having an increasing diameter.

Pulling of the cable 5a of the treatment tool 5 is all that is needed to fix the treatment tool 5 to the sheath 4, as can be seen from Fig. 21(b). As the cable 5a is pulled, it causes the diameter-increasing portion 51b to come into contact with the convexity 4b of the sheath 4. As the cable 5a is further pulled, it causes the convexity 4b to increase in diameter in association with the enlargement of the diameter-increasing portion 51b. As the convexity 4b goes over the maximal portion 51c, it causes the convexity 4b to be fitted in the concavity 51a for fixation of the treatment tool 5 to the sheath 4. It is then preferable that the opening in the sheath 4 is covered up by the lid 51d because ingress of foreign matter into the sheath 4 is held back. Letting out the cable 5a of the treatment tool 5 is all that is needed to make the treatment tool 5 movable with respect to the sheath 4. Note here that the sheath 4 may have a concavity while the treatment tool 5 may have a convexity.

Such simple structure enables the treatment tool 5 to be temporality fixed to the sheath 4, resulting in an improved operability.

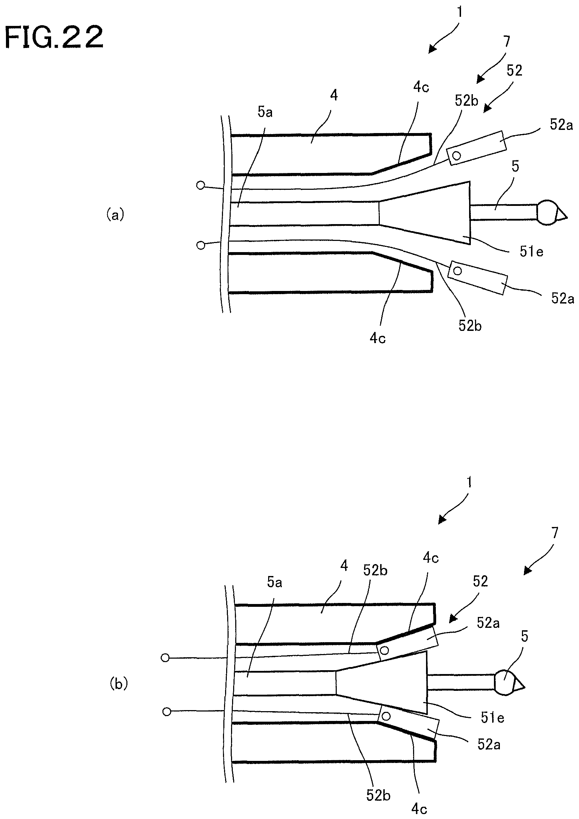

Fig. 22 is illustrative in schematic of the second example of the fixation mechanism 7 for temporal fixation of the treatment tool 5 to the sheath 4 in the embodiment described herein.

In the second example of the fixation mechanism 7, the sheath 4 includes a tapered portion 4c at its inner circumferential end, as can be seen from Fig. 22(a). The treatment tool 5 includes a diameter-increasing portion 51e whose diameter increases as it comes close to the distal end. Further, the second example of the fixation mechanism 7 includes a sandwiched portion 52a held between the sheath 4 and the treatment tool 5. The sandwiched portion 52 includes a sandwiched member 52a held between the sheath 4 and the treatment tool 5, and a coupler member 52b coupled at one end to the sandwiched member 52a. The other end of the coupler member 52b is coupled to an operating or driving section (not shown) such that it can be pushed or pulled.

Pulling of the cable 5a through the treatment tool 5 simultaneously with pulling of the coupler member 52b of the sandwiched portion 52 is all that is needed to fix the treatment tool 5 to the sheath 4, as can be seen from Fig. 22(b). As the cable 5a and coupler member 52b are pulled, it causes the sandwiched portion 52a to be held between the tapered portion 4c of the sheath 4 and the diameter-increasing portion 51e of the treatment tool 5 for fixation of the treatment tool 5 to the sheath 4. The opening in the sheath 4 is then covered up by the diameter-increasing portion 51e and sandwiched portion 52a of the treatment tool 5 for prevention of ingress of foreign matter into the sheath 4. Letting of the cable 5a and coupler member 52 out of the treatment tool 5 is all that is needed to make the treatment tool 5 movable with respect to the sheath 4.

Such simple structure enables the treatment tool 5 to be temporality fixed to the sheath 4, resulting in an improved operability.

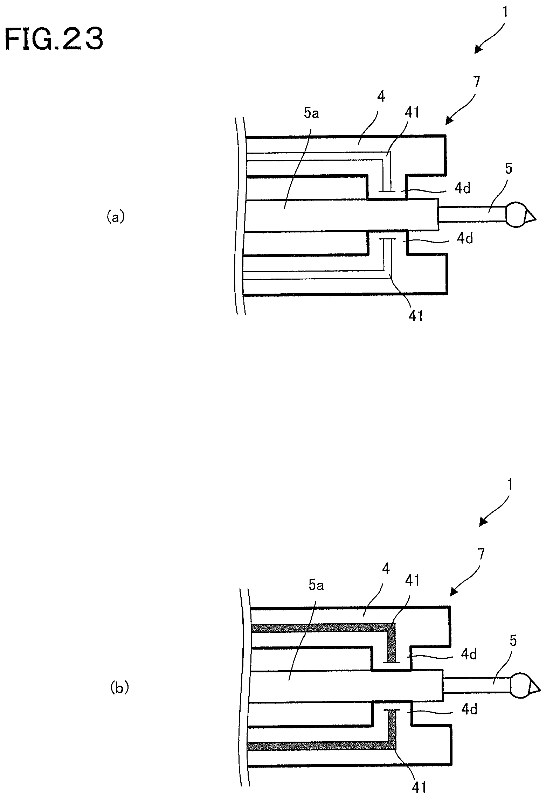

Fig. 23 is illustrative in schematic of the third example of the fixation mechanism 7 for temporal fixation of the treatment tool 5 to the sheath 4 in the embodiment described herein.

In the third example of the fixation mechanism 7, at least one set of adjoining portions 4d extends toward the inner circumferential side of the sheath 4, and is located in adjoining opposite positions with the cable 5a of the treatment tool 5 held between them, as can be seen from Fig. 23(a). There is also a flow passage 41 extending into the adjoining portions 4d in the sheath 4.

To fix the treatment tool 5 to the sheath 4, a fluid within the flow passage 4 is placed in a high pressure state, as can be seen from Fig. 23(b). The fluid may have been placed in the flow passage 41 beforehand. As the pressure of the fluid within the flow passage 42 gets high, it causes the sheath 4 formed of a flexible material to be pressed and the cable 5a to be held from both sides for fixation of the treatment tool 5 to the sheath 4. Lowering of the pressure of the fluid within the flow passage 41 is all that is needed to make the treatment tool 5 movable with respect to the sheath 4.

Such simple structure enables the treatment tool 5 to be temporality fixed to the sheath 4, resulting in an improved operability.

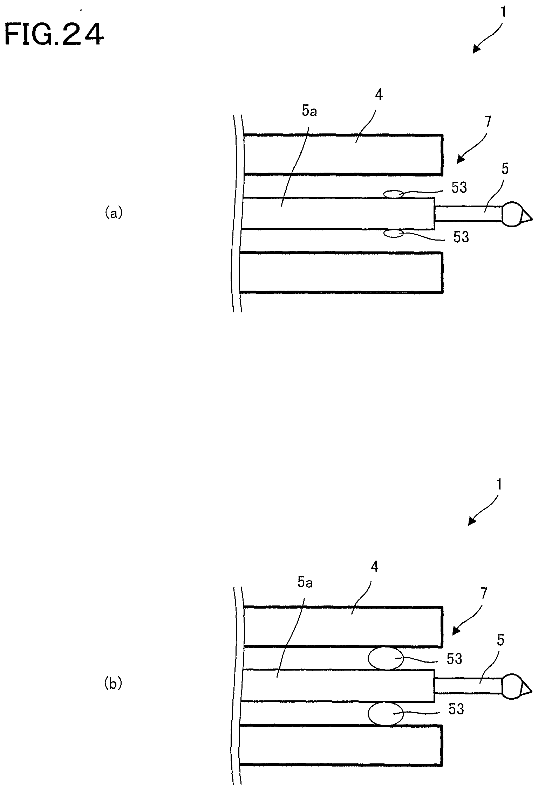

Fig. 24 is illustrative in schematic of the fourth example of the fixation mechanism 7 for temporal fixation of the treatment tool 5 to the sheath 4 in the embodiment described herein.

In the fourth example of the fixation mechanism 7, a swelling portion 53 is attached to the outer circumference of the cable 5a through the treatment tool 5, as can be seen from Fig. 24(a). The swelling portion 53 is normally attached to the cable 5a in such a way as to be movable between the cable 5a and the sheath 4.

To fix the treatment tool 5 to the sheath 4, a fluid such as air or gas is fed into the swelling portion 53, as can be seen from Fig. 24(b). As the fluid flows in, it causes the swelling portion 53 to swell out enough to press the sheath 4 for fixation of the treatment tool 5 to the sheath 4. A discharge of the fluid out of the swelling portion 53 is all that is needed to make the treatment tool 5 movable with respect to the sheath 4.

It is here to be noted that the swelling portion 53 may be attached to the inner circumferential side of the sheath 4. In this case, as the swelling portion 53 swells out, it causes the cable 5a to be held from both its sides for fixation of the treatment tool 5 to the sheath 4. A discharge of the fluid out of the swelling portion 53 is all that is needed to make the treatment tool 5 movable with respect to the sheath 4.

Such simple structure enables the treatment tool 5 to be temporality fixed to the sheath 4, resulting in an improved operability.

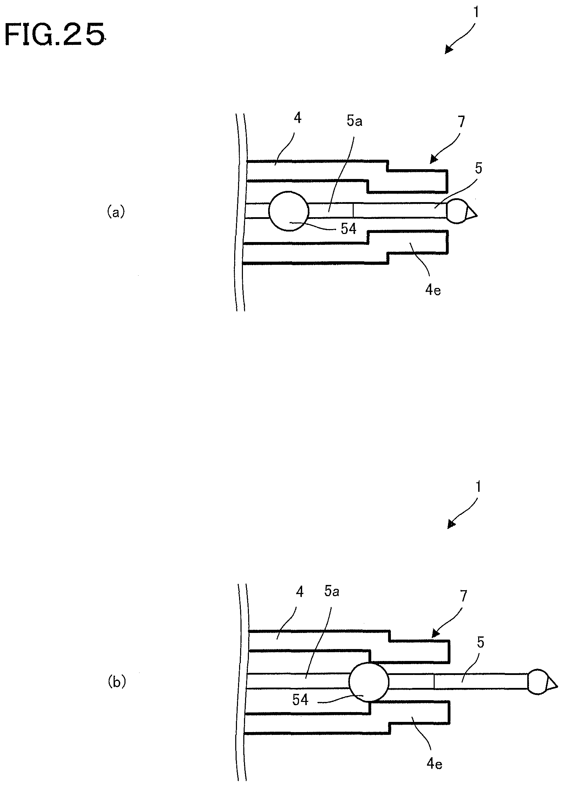

Fig. 25 is illustrative in schematic of the fifth example of the fixation mechanism 7 for temporal fixation of the treatment tool 5 to the sheath 4 in the embodiment described herein.

In the fifth example of the fixation mechanism 7, the sheath 4 includes a diameter-reduced portion 4e on the inner circumferential side of its end, as can be seen from

Fig. 25(a). The treatment tool 5 includes an enlarged portion 54 that is diametrically larger than the inner diameter of the diameter-reduced portion 4e formed on the cable 5a.

Letting of the cable 5a out of the treatment tool 5 is all that is needed to fix the treatment tool 5 to the sheath 4, as can be seen from Fig. 25(b). As the cable 5a is let out, it causes the enlarged portion 54 to be fitted over the diameter-reduced portion 4e so that the treatment tool 5 is fixed to the sheath 4 while extending out of the sheath 4. It is then preferable that the diameter-reduced portion 4e of the sheath 4 is covered up by the enlarged portion 54 because ingress of foreign matter into the sheath 4 is held back. Pulling the cable 5a of the treatment tool 5 to release the enlarged portion 54 out from the diameter-reduced portion 4e is all that is needed to make the treatment tool 5 movable with respect to the sheath 4.

Such simple structure enables the treatment tool 5 to be temporality fixed to the sheath 4, resulting in an improved operability.

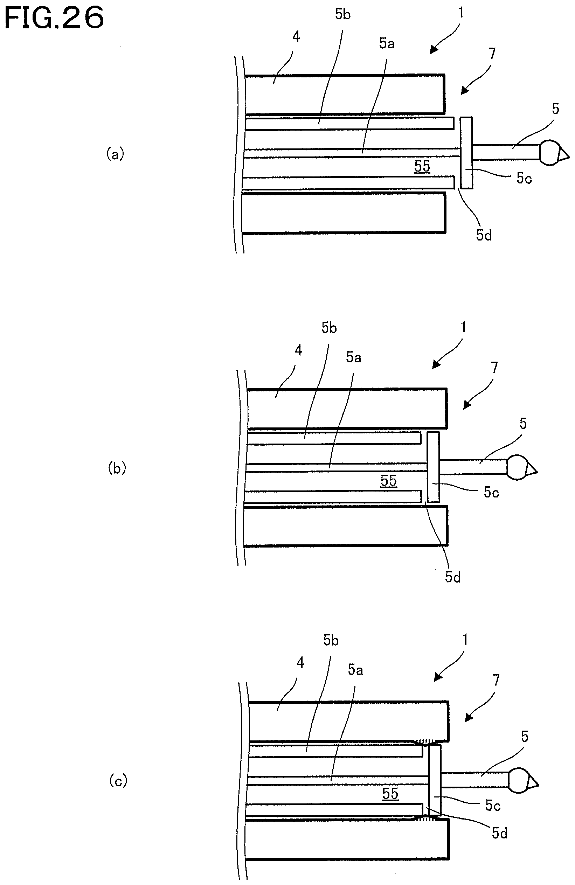

Fig. 26 is illustrative in schematic of the sixth example of the fixation mechanism 7 for temporal fixation of the treatment tool 5 to the sheath 4 in the embodiment described herein.

In the sixth example of the fixation mechanism 7, as can be seen from Fig. 26(a), a tubular member 5b is provided in such a way as to surround the operating cable 5a of the treatment tool 5. One end of the tubular member 5b is covered up by a lid 5c, and a hole 5d is formed through the tubular member 5b. The other end of the tubular member 5b is connected with a suction device (not shown) capable of sucking air on the inner circumferential side of the tubular member 5b so that there is a flow passage 55 formed on the inner circumferential side of the tubular member 5b. As can be seen from Fig. 26(b), the treatment tool 5 is stored in the sheath 4 by pulling the cable 5a or tubular member 5b of the treatment tool 5.

Fixation of the treatment tool 5 to the sheath 4 may be achieved by pulling the cable 5a or tubular member 5b of the treatment tool 5, and using the suction device (not shown) to suck air in the flow passage 55 while the treatment tool 5 remains stored in the sheath 4, as can be seen from Fig. 26(c). The sucked air sucks the sheath 4 at a place corresponding to the hole 5d. The sheath 4 comes into contact with the tubular member 5b of the treatment tool 5 to close up the hole 5d, and is fixed by suction force in place. Making the treatment tool 5 movable to the sheath 4 may be achieved by stopping the suction of air in the flow passage 55 and letting out the cable 5a or tubular member 5b of the treatment tool 5.

Such simple structure enables the treatment tool 5 to be temporality fixed to the sheath 4, resulting in an improved operability.

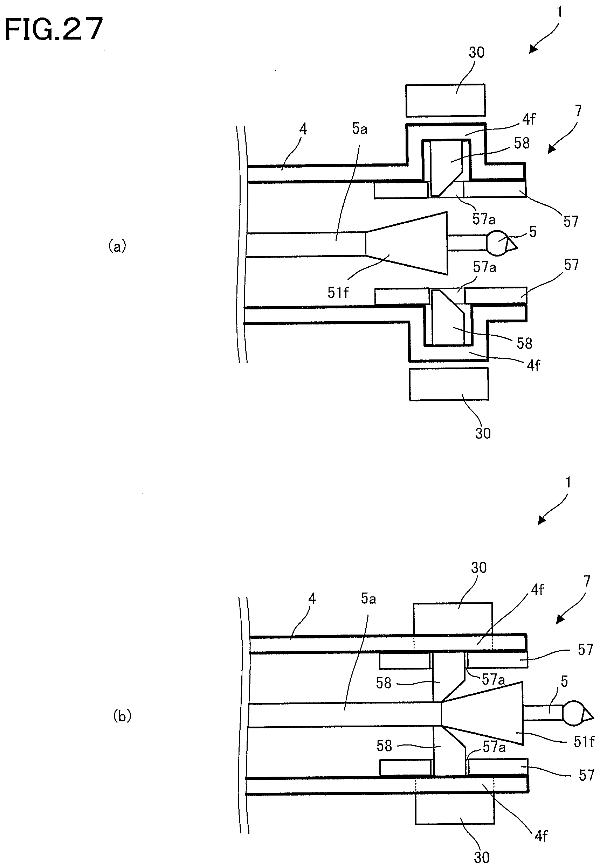

Fig. 27 is illustrative in schematic of the seventh example of the fixation mechanism 7 for temporal fixation of the treatment tool 5 to the sheath 4 in the embodiment described herein.

In the seventh example of the fixation mechanism 7, as can be seen from Fig. 27(a), the cable 5a of the treatment tool 5 is provided with a diameter-increasing portion 51f increasing in diameter toward its distal end. The sheath 4 is provided with at least one set of outwardly extending opposite protrusions 4f. On the inner circumferential side of the sheath 4 including the protrusions 4f, there are annular retaining members 57 provided, and the protrusions 4f each include an pressing member 58 on its inner circumferential side. The retaining member 57 is provided with a hole 57a through which the pressing member 58 can be inserted. Note here that the distal end portion of the pressing member 58 may be tilted in conformity with the tilt of the diameter-increasing portion 51f to facilitate movement of the treatment tool 5.

To fix the treatment tool 5 to the sheath 4, as can be seen from Fig. 27(b), the protrusions 4f of the sheath 4 are grasped by the end effector 30. As the protrusions 4f are grasped by the end effector 30, it causes the pressing member 58 to extend out from the hole 57a formed in the retaining member 57, pressing the diameter-increasing portion 51f of the treatment tool 5. As the pressing member 58 presses the diameter-increasing portion 51f, it causes the treatment tool 5 to extend out from the distal end of the sheath 4 along the tilt of the diameter-increasing portion 51f. The end effector 30 has a grasp of the retaining member 57 via the sheath 4. Then, the cable 5a is sandwiched between the pressing members 58 for fixation of the treatment tool 5 to the sheath 4.

Such simple structure enables the treatment tool 5 to be temporality fixed to the sheath 4, resulting in an improved operability.

Preferably in the fixation mechanism 7, at least a portion of each of the sheath 4, treatment tool 5 and pressing members 58 is made up of an electrically conductive wire or the like so that electric current can be passed through them in the state of Fig. 27(b). As an example, the passage of electric current may be achieved by the operation of a switch on the operating section (not shown). A local injection needle or the like mounted on the treatment tool 5 may be moved advanceably and retractably by such passage of electric current as shown in Fig. 27(b).

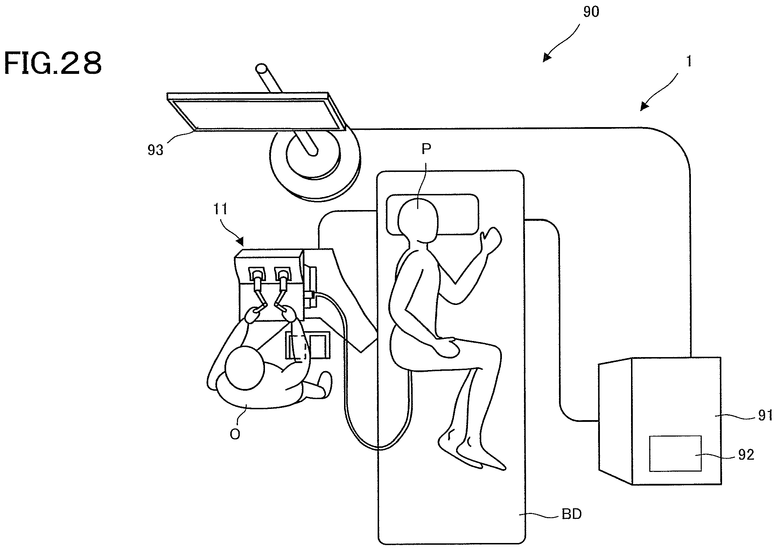

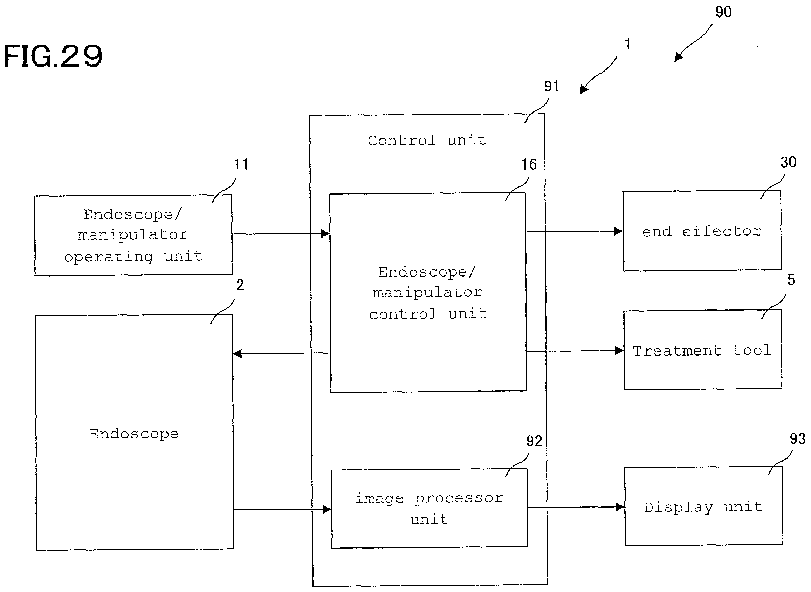

Fig. 28 is illustrative of a medical system 90 to which the medical apparatus 1 according to the embodiment described herein is applied, and Fig. 29 is illustrative in architecture of the medical system 90 to which the medical apparatus 1 according to the embodiment described herein is applied.

The medical system 90 according to the embodiment described herein incorporates the medical apparatus 1 according to the embodiment described herein. The medical system 90 includes the medical apparatus 1 including an endoscope/manipulator operating unit 11 operated by an operator O as well as an endoscope 2 and a manipulator 3 (shown in Fig. 1) that is capable of insertion through the body of a patient P lying on an operating table BD, for instance, a soft organ like the large intestine, a system control unit 91 for control of the medical apparatus 1, and a display unit 93 for displaying images acquired through the endoscope 2.

As shown in Fig. 28, the endoscope/manipulator operating unit 11 includes a pair of operating handles mounted on an operating table, and a footswitch or the like mounted on the face of the floor. The endoscope/ manipulator operating unit 11 may include a multi-joint structure. The angle of the endoscope/ manipulator operating unit 11 in operation is acquired from an angle acquisition unit such as an encoder, and in response to the acquired signals, the system control unit 91 puts an end effector 30, treatment tool 5, etc. in actuation by way of a driver, as shown in Fig. 29. Note here that the endoscope/manipulator control unit 16 shown in Fig. 1 may be installed in the medical apparatus 1 to put the end effector 30, treatment tool 5 or the like in actuation.

Images acquired through the endoscope 2 are produced out to an image processor unit 92 in the system control unit 91. The images processed in the image processor unit 92 are displayed on the display unit 93, and the operator O operates the manipulator 3 while viewing the images displayed on the display unit 93.

According to such medical system 90, it is possible to display unerring images called for by the operator, and for the operator to achieve more unerring actuation of the endoscope 2 and manipulator 3 as circumstances demand, resulting in an improved operability.

As described above, the medical apparatus 1 according to one embodiment of the invention includes the endoscope 2, the manipulator 3 having at least one degree of freedom, the flexible cylindrical sheath 4 mounted on the outer circumference of the manipulator 3, the treatment tool 5 inserted through the sheath 4, and the endoscope/ manipulator operating unit 11 for operating the endoscope 2 and manipulator 3 independently. As compared with an arrangement wherein the sheath 4 is attached to the endoscope 2, therefore, the treatment tool 5 can more easily be placed within the field of view of the endoscope 2 by mounting the sheath 4 having the treatment tool 5 inserted through it on the manipulator 3 for operation of the endoscope 2 or manipulator 3, thereby improving on operability.

The medical apparatus 1 according to one embodiment of the invention includes the endoscope/manipulator control unit 16 for independent control of the endoscope 2 and manipulator 3, wherein the endoscope/manipulator operating unit 11 includes an endoscope operating unit 12 and a manipulator operating unit 13 separately, and the endoscope/manipulator control unit 16 includes an endoscope control unit 17 and a manipulator control unit 18 separately. Thus, the medical apparatus 1 here is compatible with independent operation of the endoscope 2 and manipulator 3, resulting in a more improved operability.

The medical apparatus 1 according to one embodiment of the invention includes a first connector member 61 for connecting together the manipulator 3 and sheath 4, and a second connector member 62 located adjacent to the first connector member 61 for connecting together the manipulator 3 and sheath 4, wherein the length of the sheath 4 located between the adjoining first and second connector members 61 and 62 is longer than the length of the manipulator 3 between the adjoining first and second connector members 61 and 62 in the axial direction of the manipulator 3. Thus, the sheath 4 between the adjoining first and second connector members 61 and 62 has a play enough to provide smooth bending of the manipulator 3 between the adjoining first and second connector members 61 and 62 without any interference of the sheath 4 with the bending movement of the manipulator 3, resulting in an improved operability.

According to the medical apparatus 1 according to one embodiment of the invention, the first 61 or the second connector member 62 connects together the manipulator 3 and sheath 4 such that they are relatively movable. Upon bending or curving of the manipulator 3, therefore, the sheath 4 can move to a position where no active load is applied so that even with disturbance such as friction, the manipulator 3 can be smoothly actuated.

The medical apparatus 1 according to one embodiment of the invention includes the movement restrictors 38 and 39 for stopping movement of the first 61 or the second connector member 62 in a given position for movement restriction. It is thus possible to stop movement of the connector member 6 with respect to the manipulator 3. For instance, it is possible to facilitate insertion of the cable 5a through the sheath 4.

The medical apparatus 1 according to one embodiment of the invention includes the fixation mechanism 7 for temporal fixation in a given position of the treatment tool 5 which can move forward and backward into the sheath 4. It is thus possible to improve on the operability of the treatment tool 5 during use in a fixed state.

In the medical apparatus 1 according to one embodiment of the invention, the manipulator 3 includes the end effector 30 for implementing medical treatments, resulting in a decrease in the number of replacement of the treatment tool 5 and a curtailment of the time taken for replacement. It is also possible to operate both the end effector 30 and the manipulator 5 by means of the single manipulator 3. It is thus possible to improve on the operability of the manipulator 3.

In the medical apparatus 1 according to one embodiment of the invention, the treatment tool 5 and end effector 30 can be operated in an independent manner so that the medical apparatus 1 can be well compatible with each treatment, resulting in an improved operability.

In the medical apparatus 1 according to one embodiment of the invention, the treatment tool 5 can be temporality fixed to the manipulator 3 or the manipulator 3 can be temporality fixed to the treatment tool 5 so that the treatment tool 5 can be actuated in a stable manner. It is also possible to reduce interference of the treatment tool 5 with other endoscope or manipulator.

The medical system 90 according to one embodiment of the invention includes the system control unit 91 for control of the medical apparatus 1 and the display unit 93 for displaying images acquired through the endoscope 2. The system control unit 91 enables images obtained through the endoscope 2 to be shown on the display unit 93 so that it is possible for the operator to actuate the endoscope 2 and manipulator 3 more unerringly as circumstances demand, resulting in a more improved operability.

It is here to be appreciated that the invention is in no sense limited to such embodiments as described. While the explanation of some embodiments embraces numerous specific details for illustration, it would be obvious to those skilled in the art that diverse variations or modifications made thereto are included within the scope of the invention. In other words, illustrative embodiments of the invention are described without ridding the claimed inventions of generality and imposing any limitation thereon.

### Reference Signs List

- 1:: Medical apparatus
- 2:: Endoscope
- 3:: Manipulator
- 4:: Sheath
- 5:: Treatment tool
- 6:: Connector
- 7:: Fixation mechanism
- 90:: Medical system
- 91:: System control unit
- 92:: Image processor unit
- 93:: Display unit

## Claims

1. A medical apparatus, **characterized by** comprising:
an endoscope;
a manipulator having at least one degree of freedom;
a flexible cylindrical sheath mounted on an outer circumference of the manipulator;
a treatment tool inserted through the sheath;
and
an endoscope/manipulator operating unit for independent operation of the endoscope and the manipulator.

2. A medical apparatus according to claim 1, further comprising
an endoscope/manipulator control unit for independent control of the endoscope and the manipulator,
wherein
the endoscope/manipulator operating unit includes an endoscope operating unit and a manipulator operating unit separately,
and
the endoscope/manipulator control unit includes an endoscope control unit and a manipulator control unit separately.

3. A medical apparatus according to claim 1 or 2, further comprising:
a first connector member for connecting together the manipulator and the sheath;
and
a second connector member located adjacent to the first connector member for connecting together the manipulator and the sheath,
wherein a length of the sheath located between adjoining the first connector member and the second connector member is longer than a length of the manipulator between adjoining the first connector member and the second connector member along an axial direction of the manipulator.

4. A medical apparatus according to claim 3,
wherein the first connector member or the second connector member connects together the manipulator and the sheath such that they are relatively movable.

5. A medical apparatus according to claim 4,
further comprising a movement restrictor for stopping movement of the first connector member or the second connector member in a given position for movement restriction.

6. A medical apparatus according to any one of claims 1 to 5,
further comprising a fixation mechanism for temporal fixation in a given position of the treatment tool which can move forward and backward into the sheath.

7. A medical apparatus according to any one of claims 1 to 6,
wherein the manipulator includes an end effector for implementing medical treatments.

8. A medical apparatus according to claim 7, wherein the treatment tool and the end effector are capable of independent operation.

9. A medical apparatus according to any one of claims 1 to 8,
wherein
the treatment tool is capable of fixing the manipulator temporarily in place,
and
the manipulator is capable of fixing the treatment tool temporarily in place.

10. A medical system, **characterized by** comprising:
a system control unit for control of a medical apparatus;
and
a display unit for displaying images acquired through the endoscope,
wherein the system control unit enables images acquired through the endoscope to be shown on the display unit.
